# EUROPEAN PATENT APPLICATION

(11) **EP 3 795 583 A1**
(43) Date of publication of application: **24.03.2021**
(21) Application number: 19198358.4
(22) Date of filing: 19.09.2019
(51) Int. Cl.: C07K 14/54, A61K 38/20, A61P 35/00

(54) **IL10/FC FUSION PROTEINS USEFUL AS ENHANCERS OF IMMUNOTHERAPIES**

(71) Applicant: Ecole Polytechnique Fédérale de Lausanne (EPFL), 1015 Lausanne (CH)
(72) Inventor: GUO, Yugang, 1025 St-Sulpice (CH); TANG, Li, 1112 Echichens (CH); XIE, Yu-Qing, 1024 Ecublens (CH)
(74) Representative: reuteler & cie SA

(57) **Abstract**

The present invention relates to new agents useful for anti-cancer therapy such as anti-cancer adoptive T-cell transfer (ACT) immunotherapy or immune check-point blockade therapy and related compositions, uses and methods thereof.

SEQUENCE LISTING

<110>  ECOLE POLYTECHNIQUE FEDERALE DE LAUSANNE (EPFL)

<120>  IL10/FC FUSION PROTEINS USEFUL AS ENHANCERS OF IMMUNOTHERAPIES

<130>  P2401EP00

<160>  4

<170>  PatentIn version 3.5

<210>  1
<211>  160
<212>  PRT
<213>  Homo sapiens

<400>  1

Ser Pro Gly Gln Gly Thr Gln Ser Glu Asn Ser Cys Thr His Phe Pro
1               5                   10                  15

Gly Asn Leu Pro Asn Met Leu Arg Asp Leu Arg Asp Ala Phe Ser Arg
            20                  25                  30

Val Lys Thr Phe Phe Gln Met Lys Asp Gln Leu Asp Asn Leu Leu Leu
        35                  40                  45

Lys Glu Ser Leu Leu Glu Asp Phe Lys Gly Tyr Leu Gly Cys Gln Ala
    50                  55                  60

Leu Ser Glu Met Ile Gln Phe Tyr Leu Glu Glu Val Met Pro Gln Ala
65                  70                  75                  80

Glu Asn Gln Asp Pro Asp Ile Lys Ala His Val Asn Ser Leu Gly Glu
                85                  90                  95

Asn Leu Lys Thr Leu Arg Leu Arg Leu Arg Arg Cys His Arg Phe Leu
            100                 105                 110

Pro Cys Glu Asn Lys Ser Lys Ala Val Glu Gln Val Lys Asn Ala Phe
        115                 120                 125

Asn Lys Leu Gln Glu Lys Gly Ile Tyr Lys Ala Met Ser Glu Phe Asp
    130                 135                 140

Ile Phe Ile Asn Tyr Ile Glu Ala Tyr Met Thr Met Lys Ile Arg Asn
145                 150                 155                 160

<210>  2
<211>  217
<212>  PRT
<213>  Artificial sequence

<220>
<223>  Non-cytolytic IgG1 Fc

<400>  2

Ala Pro Glu Val Ala Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Ser Ile Glu Lys Thr Ile Ser Lys Ala Lys Gly Gln
            100                 105                 110

Pro Arg Glu Pro Gln Val Tyr Thr Leu Pro Pro Ser Arg Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215

<210>  3

<211>  15
<212>  PRT
<213>  Artificial sequence

<220>
<223>  Flexible hinge 1

<400>  3

Glu Pro Lys Ser Cys Asp Lys Thr His Thr Cys Pro Pro Cys Pro
1               5                   10                  15


<210>  4
<211>  392
<212>  PRT
<213>  Artificial Sequence

<220>
<223>  IL-10/Fc fusion protein 1

<400>  4

Ser Pro Gly Gln Gly Thr Gln Ser Glu Asn Ser Cys Thr His Phe Pro
1               5                   10                  15


Gly Asn Leu Pro Asn Met Leu Arg Asp Leu Arg Asp Ala Phe Ser Arg
            20                  25                  30


Val Lys Thr Phe Phe Gln Met Lys Asp Gln Leu Asp Asn Leu Leu Leu
        35                  40                  45


Lys Glu Ser Leu Leu Glu Asp Phe Lys Gly Tyr Leu Gly Cys Gln Ala
    50                  55                  60


Leu Ser Glu Met Ile Gln Phe Tyr Leu Glu Glu Val Met Pro Gln Ala
65                  70                  75                  80


Glu Asn Gln Asp Pro Asp Ile Lys Ala His Val Asn Ser Leu Gly Glu
                85                  90                  95


Asn Leu Lys Thr Leu Arg Leu Arg Leu Arg Arg Cys His Arg Phe Leu
            100                 105                 110


Pro Cys Glu Asn Lys Ser Lys Ala Val Glu Gln Val Lys Asn Ala Phe
        115                 120                 125


Asn Lys Leu Gln Glu Lys Gly Ile Tyr Lys Ala Met Ser Glu Phe Asp
    130                 135                 140


Ile Phe Ile Asn Tyr Ile Glu Ala Tyr Met Thr Met Lys Ile Arg Asn
145                 150                 155                 160

Glu Pro Lys Ser Cys Asp Lys Thr His Thr Cys Pro Pro Cys Pro Ala
                165                 170                 175

Pro Glu Val Ala Gly Gly Pro Ser Val Phe Leu Phe Pro Pro Lys Pro
            180                 185                 190

Lys Asp Thr Leu Met Ile Ser Arg Thr Pro Glu Val Thr Cys Val Val
        195                 200                 205

Val Asp Val Ser His Glu Asp Pro Glu Val Lys Phe Asn Trp Tyr Val
    210                 215                 220

Asp Gly Val Glu Val His Asn Ala Lys Thr Lys Pro Arg Glu Glu Gln
225                 230                 235                 240

Tyr Asn Ser Thr Tyr Arg Val Val Ser Val Leu Thr Val Leu His Gln
                245                 250                 255

Asp Trp Leu Asn Gly Lys Glu Tyr Lys Cys Lys Val Ser Asn Lys Ala
            260                 265                 270

Leu Pro Ala Ser Ile Glu Lys Thr Ile Ser Lys Ala Lys Gly Gln Pro
        275                 280                 285

Arg Glu Pro Gln Val Tyr Thr Leu Pro Pro Ser Arg Glu Glu Met Thr
    290                 295                 300

Lys Asn Gln Val Ser Leu Thr Cys Leu Val Lys Gly Phe Tyr Pro Ser
305                 310                 315                 320

Asp Ile Ala Val Glu Trp Glu Ser Asn Gly Gln Pro Glu Asn Asn Tyr
                325                 330                 335

Lys Thr Thr Pro Pro Val Leu Asp Ser Asp Gly Ser Phe Phe Leu Tyr
            340                 345                 350

Ser Lys Leu Thr Val Asp Lys Ser Arg Trp Gln Gln Gly Asn Val Phe
        355                 360                 365

Ser Cys Ser Val Met His Glu Ala Leu His Asn His Tyr Thr Gln Lys
    370                 375                 380

Ser Leu Ser Leu Ser Pro Gly Lys
385                 390

## Description

### Field of the Invention

The present invention relates generally to the field of anti-cancer therapy, in particular co-agents useful in anti-cancer immunotherapy such as adoptive T-cell transfer (ACT) immunotherapy and immune check-point blockades.

### Background of the Invention

Adoptive T-cell transfer (ACT) immunotherapy has produced stunning clinical results recently. Unlike traditional chemo- and radiotherapy, immunotherapy actives host immune system to attack malignancies, and this potentially offers long-term protection from recurrence with less toxicity in comparison to conventional chemo- and radiation therapy. In adoptive CD8+ T cell therapy, large numbers of tumor-specific T cells are sourced from patients and expanded *in vitro* and infused back to patients (Jiang et al., 2019, Cancer Lett., 10;462:23-32*;* Levine et al., 2017 Cell Therapy. Mol. Ther. - Methods Clin. Dev. 4, 92-101*).* T cells can be expanded from naturally-induced tumor-specific CD8+ T cells isolated from tumor infiltrating lymphocytes (TILs) or genetically-modified autologous circulating CD8+ T cells. The engineered T cells expressed tumor-specific antigen receptors including chimeric antigen receptors (CARs) and T cell receptors (TCRs), prepared from cultured T cell clones, respectively. The most successful ACT, anti-CD19 chimeric antigen receptor T (CAR-T) cell therapy directed against B cell lymphoma, is already approved for use based on evidence of efficacy (June et al., 2018, Science 359, 1361-1365*).* Despite the great success in treating haematological malignancies, solid tumor remains a major challenge for ACT and cures remain rare (Lim et al., 2017, Cell 168, 724-740*; Jiang et al., 2019, supra).* It has been reported that tumor-infiltrating lymphocytes (TILs) show a gradual loss of effector functions and proliferation capacity in the tumor microenvironment (TME), defined as T cell 'exhaustion' (Thommen et al., 2018, Cancer Cell, 33, 547-562*).* From a metabolic point of view, persistent antigen stimulation and other metabolic stress in the TME greatly alters T cell signaling and impair their antitumor immune response *(*Schietinger et al., 2016, Immunity 45, 389-401*;* Vodnala et al., 2019, Science 363*).* Metabolic reprogramming of adoptively transferred T cells through *ex vivo* pre-treatment has been shown to enhance ACT immunotherapy (Li et al., 2019, Nat. Rev. Clin. Oncol., doi:10.1038/s41571-019-020)*.* Very few pre-clinical studies were performed on *in vivo* metabolic intervention of tumor infiltrating CD8+ T cells (Chamoto et al., 2017, Proc. Natl. Acad. Sci. U. S. A., 114, E761-E770*;* Chowdhury et al., 2018, Cancer Immunol. Res. 6, 1375-1387*)* but those methods were lacking specificity for the target cells Some pre-clinical studies were performed on *ex vivo* metabolic intervention of tumour antigen specific CD8+ T cells or CAR T cells (Klebanoff et al., 2004, Proc. Natl. Acad. Sci. U. S. A. 101, 1969-1974*; Vodnala et al., 2019, supra;* Klebanoff et al., 2017, JCI Insight 2*).* However, a facile and safe *in vivo* metabolic intervention that can be combined with ACT to eradicate solid tumors and induce durable cures is still lacking.

Interleukine-10 (IL-10), a member of the IL-10 family cytokines is generally considered immunosuppressive as it reduces tissue damage caused by uncontrolled inflammatory responses (Moore et al., 2001, Annu. Rev. Immunol. 19, 683-765)*.* The use of in clinic of its anti-inflammatory properties was limited by its short circulating half-life in the serum and therefore a fusion protein containing human IL-10 and an IgG Fc fragment (hIL-10/Fc) has been designed and expressed in *Pichia pastoris* (Guo et al., 2012, Protein Expr Purif., 83(2):152-6*)* to enhance the circulating half-life of human IL-10 *in vivo* and where the immune inhibitory effects have been suggested for the treatment of autoimmune disease. Therefore, there is an urgent need for safe tools for the metabolic reprogramming of adoptively transferred T cells that can be combined with ACT to induce durable cures for solid tumors.

### Summary of the Invention

The present invention is based on the unexpected findings that metabolic reprograming of tumor infiltration lymphocytes with a fusion protein IL-10/Fc markedly enhanced the efficacy of ACT against established solid tumors in syngeneic tumor bearing mouse models. It has been observed that surprisingly a fusion protein IL-10/Fc of the invention selectively expands tumor specific PD-1+TIM-3+CD8+ T cells in the tumor microenvironment, which indicates that the fusion protein had little systemic influence on other cell subsets with good safety profile compared to IL-12 or IL-15 based therapies (Wang et al., 2017, Nat. Commun., 8, 1-15*;* Momin et al., 2019, Sci. Transl. Med. 11, eaaw2614*;* Berger et al., 2009, Blood, 114, 2417-2426*,* Huntington et al., 2011, Proc. Natl. Acad. Sci. U. S. A., 108, 6217-6222*;* Guo et al. 2015, J. Immunol. 195, 2353-2364*).* It is worth noting that the observed dramatic efficacy (90% treated mice with very aggressive tumors were completely cured) of a combination of a fusion protein IL-10/Fc of the invention and ACT was totally unexpected as IL-10/Fc was typically considered as an immune-suppressive cytokine and to contribute negatively to cancer therapy.

Since there is currently no *in vivo* metabolic intervention strategy on tumor infiltrating CD8+ T cells available for highly effective ACT cancer immunotherapy, there is a great potential for the use of a fusion protein IL-10/Fc of the invention or variants thereof for use as an enhancer of immunotherapies such as ACT therapies or immune check-point blockades.

According to a first aspect, the invention provides a Fc fusion protein for use in the prevention and/or treatment of a cancer, wherein said Fc fusion protein is a polypeptide comprising an immunoglobulin IgG Fc domain and a heterologous polypeptide a comprising a sequence of a human IL-10 or a variant thereof, wherein the heterologous polypeptide is covalently linked to the N-terminus or the C-terminus of the Fc domain by a polypeptide linker (e.g. a flexible hinge).
According to another aspect of the invention, is provided a pharmaceutical composition comprising at least one Fc fusion protein according to the invention and a pharmaceutically acceptable carrier, diluent or excipient thereof and at least one agent useful in anti-cancer immunotherapy.
According to another, the invention provides a use of a Fc fusion protein according to the invention for the preparation of a pharmaceutical composition for the prevention and/or treatment of a cancer.
According to another, the invention provides a method of preventing or treating a cancer, said method comprising administering in a subject in need thereof a therapeutically effective amount of at least one Fc fusion protein of the invention.
According to another, the invention provides a method of inducing immunity or restoring of responsiveness to immunotherapy, in a subject, said method comprising administering a immune check-point blockades in combination with a Fc fusion protein of the invention in a subject in need thereof.

### Description of the figures

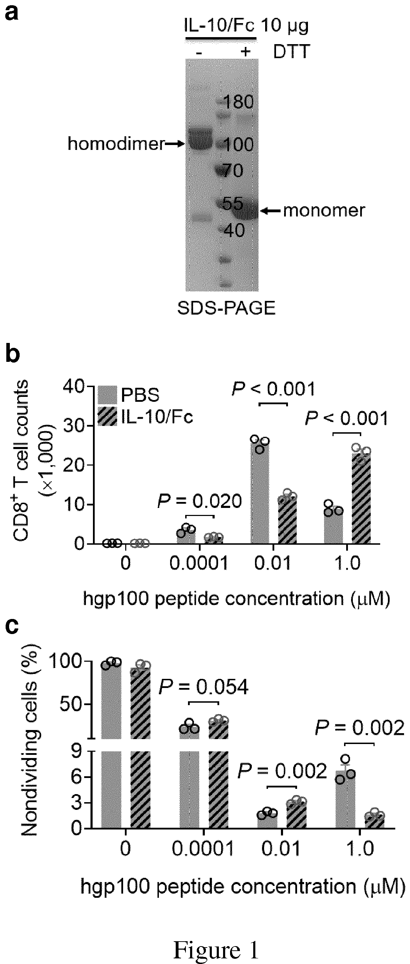

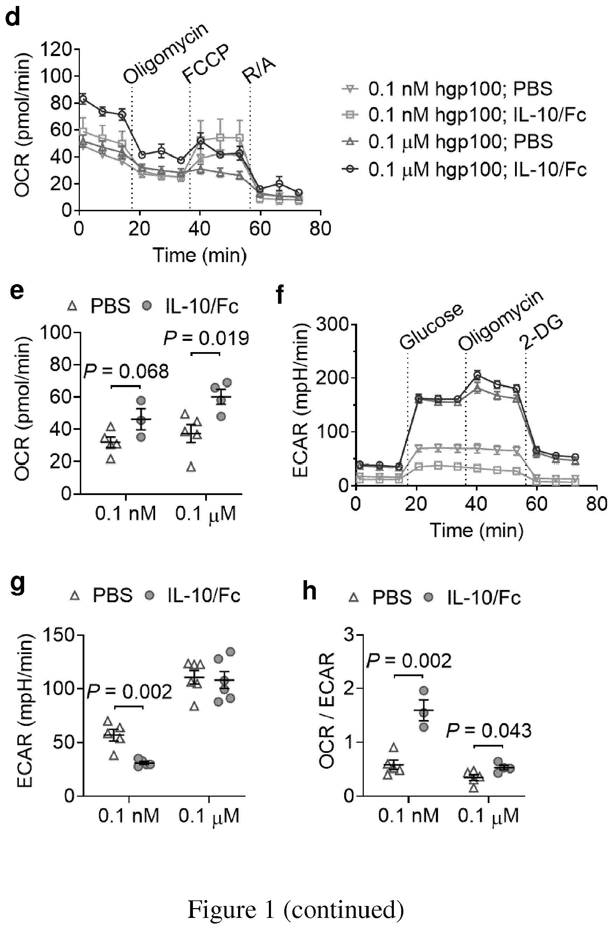

**Figure 1** represents IL-10/Fc promotes OXPHOS of CD8+ T-cells during priming phase and enhances T-cell proliferation. **(a)** SDS-PAGE (sodium dodecyl sulfate-polyacrylamide gel electrophoresis) of recombinant human IL-10 and IgG1 Fc fusion protein. Disulfide bond between each monomers was cleaved in the presence of reducing agent DTT (dithiothreitol). **(b and c)** Freshly isolated Pmel-1 mouse splenocytes were labelled with proliferation-tracking dye CFSE (carboxyfluorescein succinimidyl ester) and were stimulated with different concentrations of cognate peptide hgp100 with or without the presence of IL-10/Fc for 3 days. CD8⁺ T cell counts **(b)** and non-dividing cell percentages **(c)** were analyzed by flow cytometry. **(d)** Representative oxygen consumption rate (OCR) trace for CD8⁺ T cells stimulated as (b and c). **(e)** Statistical analysis of maximal OCR from (d). **(f)** Representative extracellular acidification rate (ECAR) trace for CD8⁺ T cells stimulated as (b and c). **(g)** Statistical analysis of maximal ECAR from (f). **(h)** Ratios of OCR/ECAR from (e) and (g). Data represent at least three independent experiments. Error bars indicate SEMs.

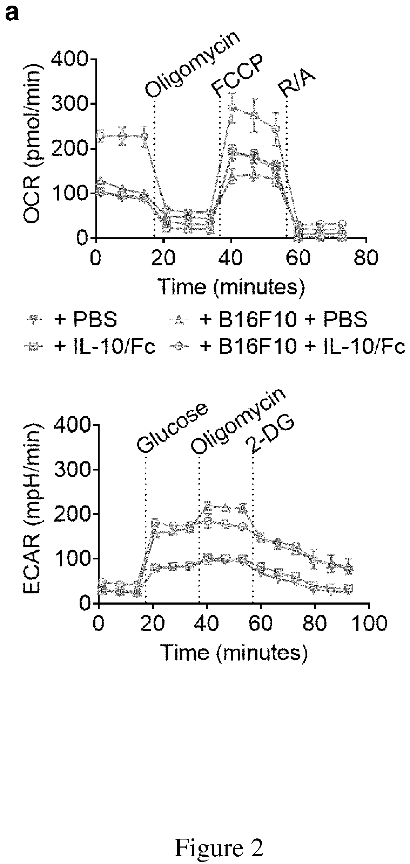

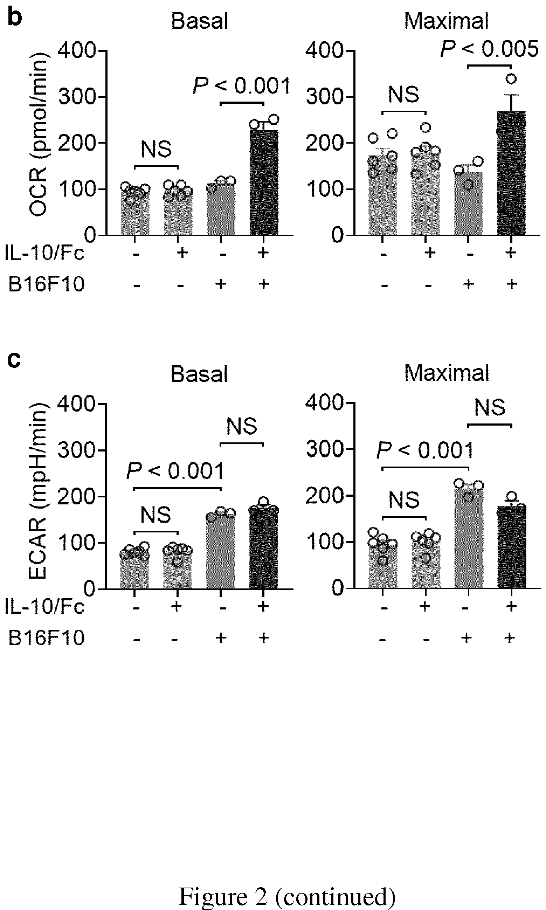

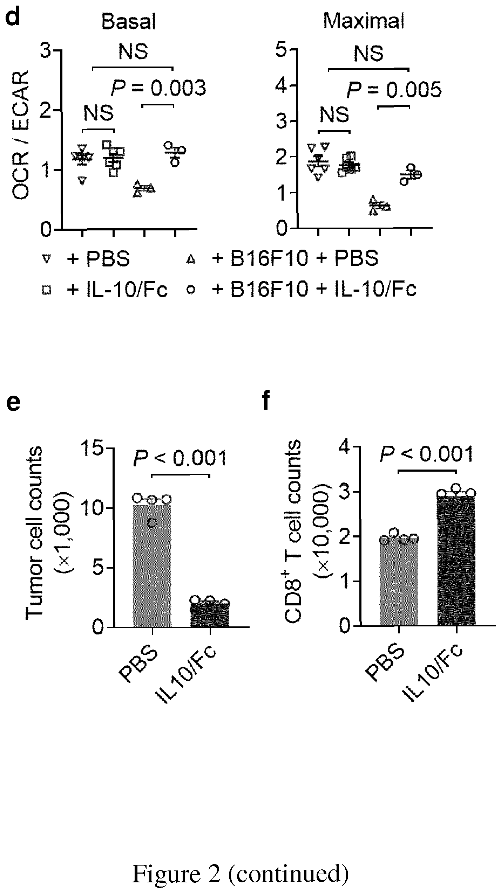

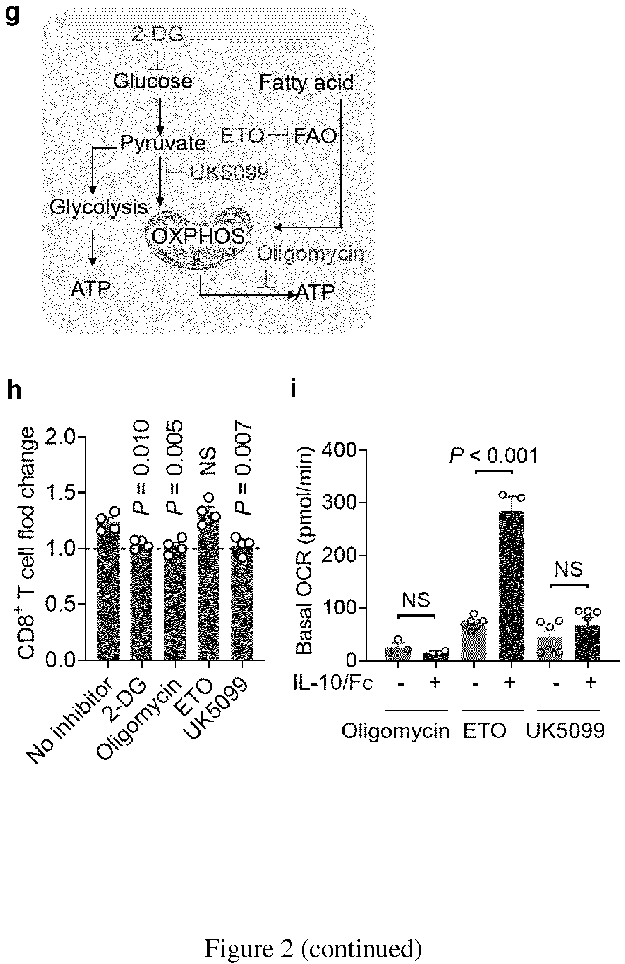

**Figure 2** represents IL-10/Fc reprograms T cells metabolism upon TCR stimulation through a pyruvate-dependent manner. **(a)** Pmel-1 mouse CD8⁺ T cells were activated by stimulation with hgp100 peptides for 3 days and rested for another 4 days, followed by co-culture with B16F10 mouse melanoma cells for further 2 days, with or without the presence of IL-10/Fc. Control Pmel-1 CD8 T cells were kept in resting phase without B16F10 cells. Representative OCR trace and representative ECAR trace for CD8⁺ T cells isolated from the co-culture. **(b)** Statistical analysis of maximal OCR from (a). **(c)** Statistical analysis of maximal ECAR from (a). **(d)** Ratios of OCR/ECAR from (c) and (d). **(e)** B16F10 tumor cell counts from (a). **(f)** CD8⁺ T cell counts from (a). **(g)** Schematic abstract of metabolism pathways and inhibitors for each part. **(h)** Pmel-1 mouse CD8⁺ T cells were activated by stimulation with hgp100 peptides for 3 days and rested for another 4 days, followed by anti-CD3 tetramers re-stimulation for further 2 days, with or without the presence of IL-10/Fc, and with the presence of different inhibitors in (g). CD8⁺ T cell fold change represents the cell counts with IL-10/Fc versus without IL-10/Fc. **(i)** Basal OCR of CD8⁺ T cell from (h). Data represent at least three independent experiments. Error bars indicate SEMs.

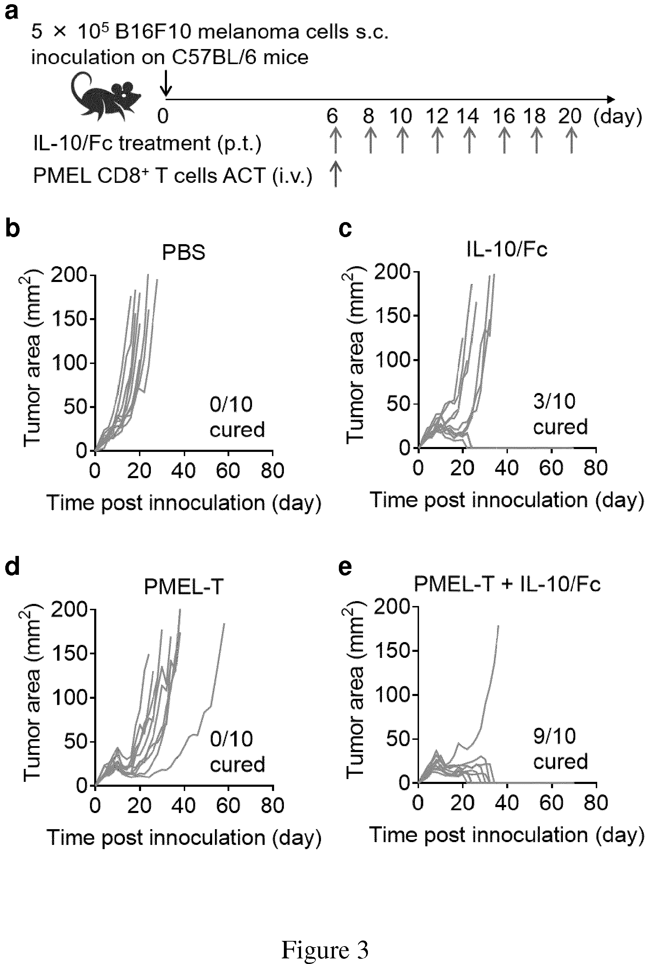

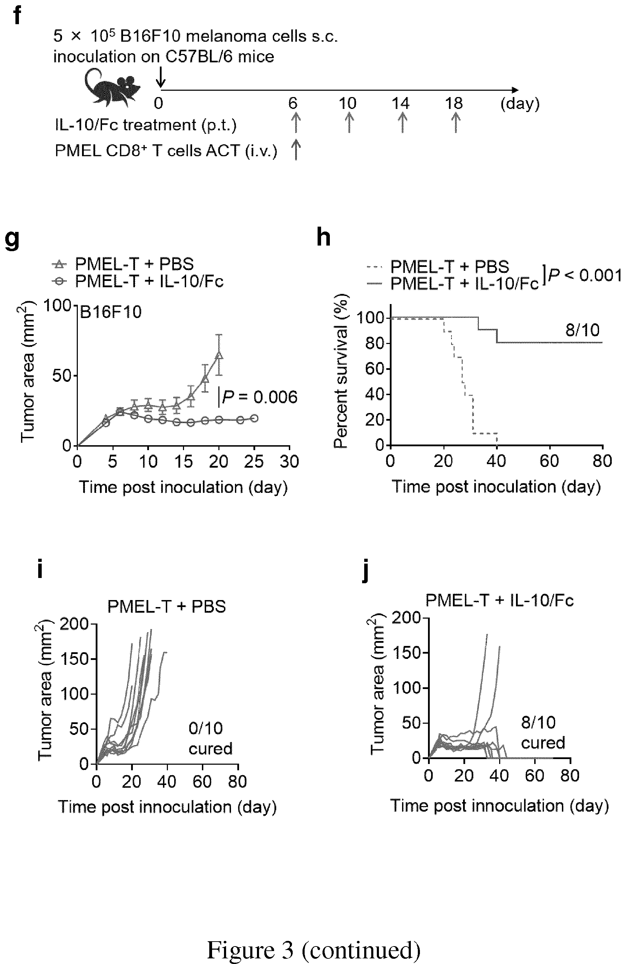

**Figure 3** represents IL-10/Fc potentiates adoptive T cells therapy to eradicate established highly aggressive mouse melanoma tumors. **(a)** Experimental scheme for Pmel CD8⁺ T cells ACT and IL-10/Fc combination therapy for mouse B16F10 melanoma model. **(b-e)** Individual tumor growth curves of PBS control group **(b),** IL-10/Fc monotherapy **(c),** ACT monotherapy **(d)** and combination therapy **(e)** mice treated as in (a). **(f)** Experimental scheme for Pmel CD8⁺ T cells ACT and IL-10/Fc combination therapy for mouse B16F10 melanoma model with reduced doses. **(g)** Tumor growth was monitored for mice treated as in (f). **(h)** Survival plot of mice treated as in (f). **(i and j)** Individual tumor growth curves of ACT monotherapy group **(i)** and combination therapy **(j)** mice treated as in (f). Data represent at least three independent experiments. Error bars indicate SEMs.

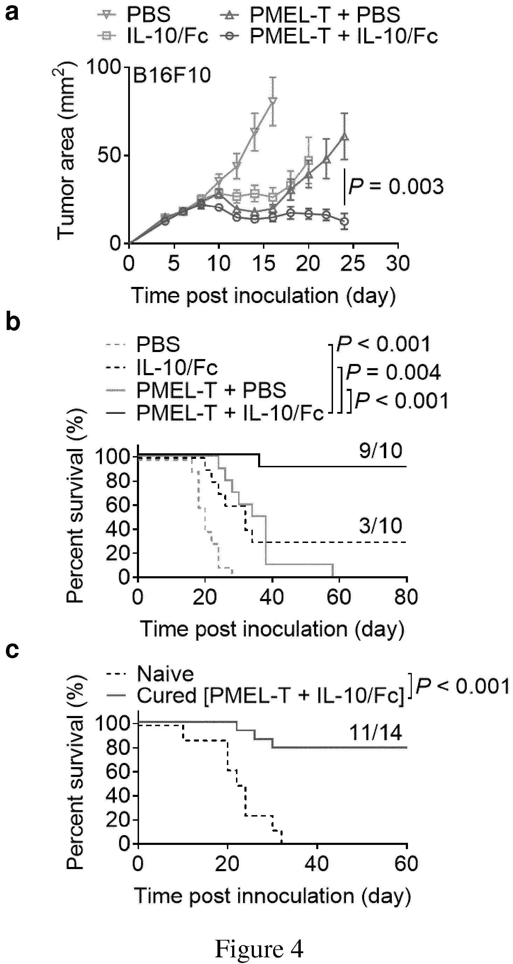

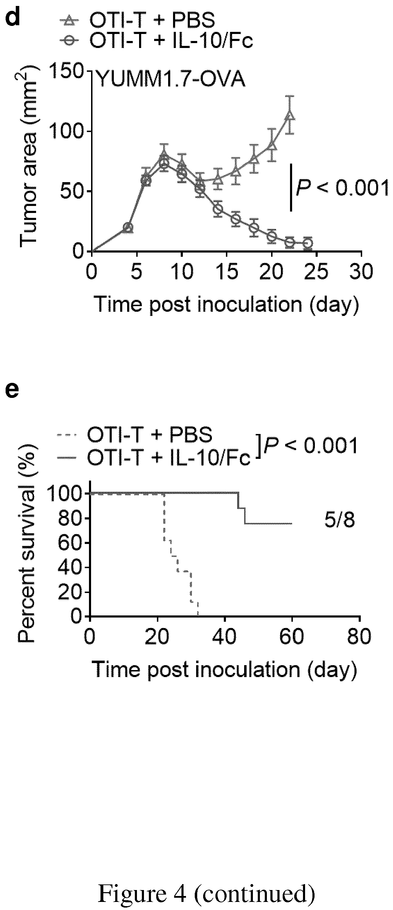

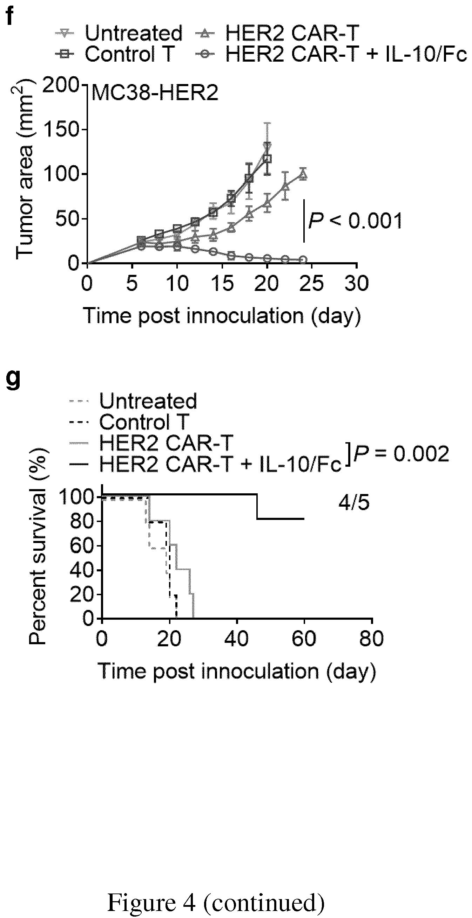

**Figure 4** represents IL-10/Fc combines with adoptive T cells therapy to eradicate established tumors in multiple syngeneic tumor mouse models. **(a)** Tumor growth was monitored for mice treated as in Fig 3(a). **(b)** Survival plot of mice treated as in Fig 3(a). **(c)** Survived mice from combination group as in Fig 3(a) were re-challenged with 1 x 10⁵ B16F10 cells subcutaneously at day 90 post primary inoculation. Naive wild type mice were inoculated with same number of tumor cells as controls. Survival plot of mice was monitored. **(d)** 1 x 10⁶ YUMM1.7-OVA mouse melanoma cells were subcutaneously inoculated on C57BL/6J mice. OVA-specific OT-I CD8⁺ T cells were applied as ACT in this model. Experimental schedule was same as in Fig 3(a.) Tumor growth was monitored. (e) Survival plot of mice treated as in (d). **(f)** 1 x 10⁶ MC38-HER2 mouse colon cancer cells were subcutaneously inoculated on C57BL/6J mice. HER2-CAR-T cells were applied as ACT in this model. Experimental schedule was same as in Fig 3(a.) Tumor growth was monitored. **(g)** Survival plot of mice treated as in (f). Data represent at least three independent experiments for (a) and (b). Data from (c) are the collection of two independent experiments. Error bars indicate SEMs.

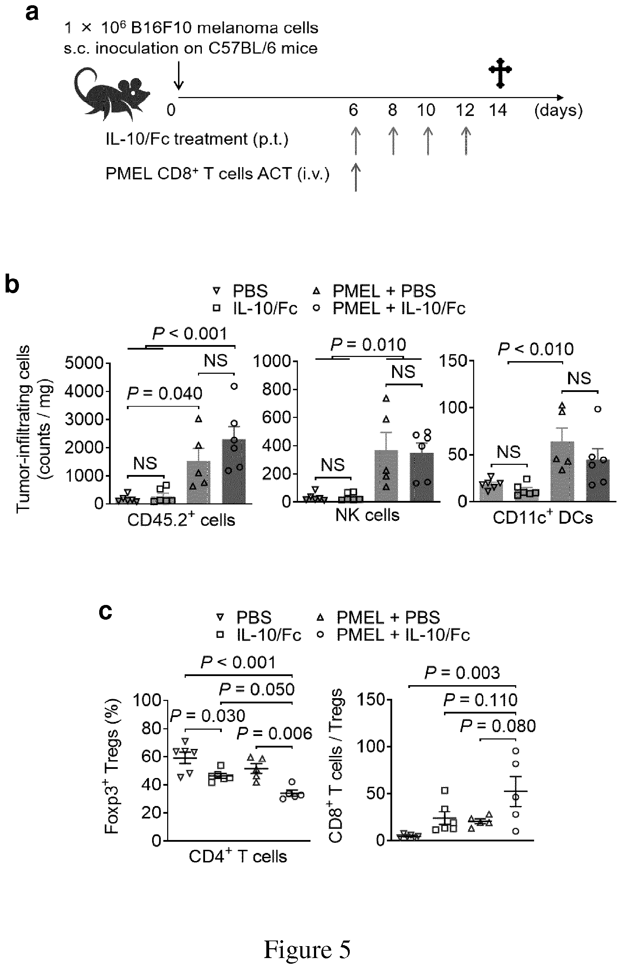

**Figure 5** shows IL-10/Fc enhances anti-tumor immunity. **(a)** Experimental scheme for flow cytometry mechanism study in mouse B16F10 melanoma model. Mice were sacrificed on day 14 and TILs were analyzed by flow cytometry. **(b)** Total CD45.2⁺ leukocytes and NK cells and CD11c⁺ dendritic cells (DCs) density in tumor. **(c)** Foxp3⁺ regulatory T cells (Tregs) percentages in total CD4⁺ T cells and the ratios of total CD8⁺ T cell number to the Treg cell number. Data represent at least three independent experiments. Error bars indicate SEMs.

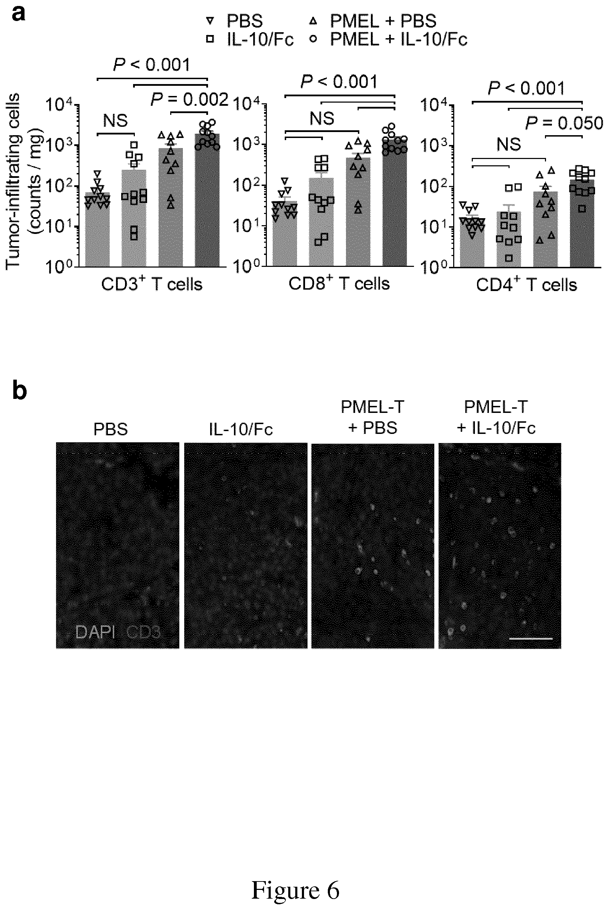

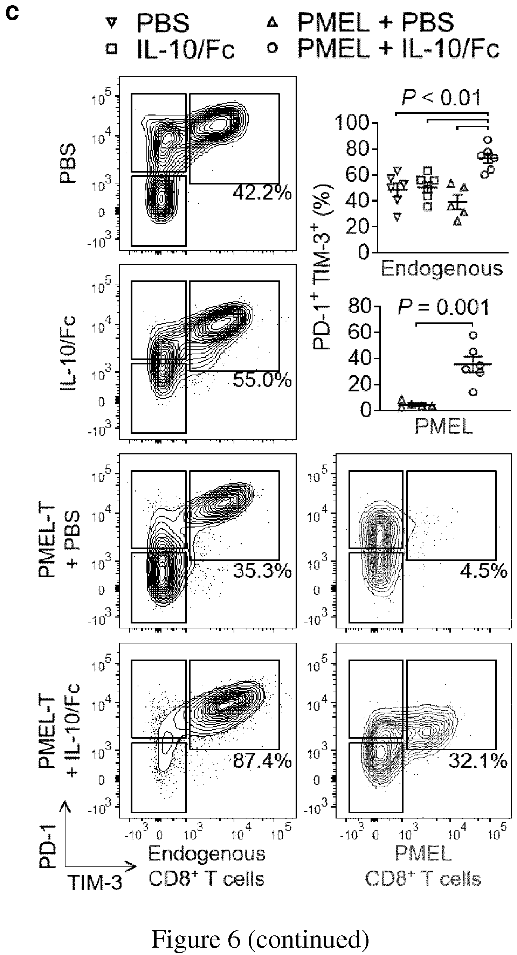

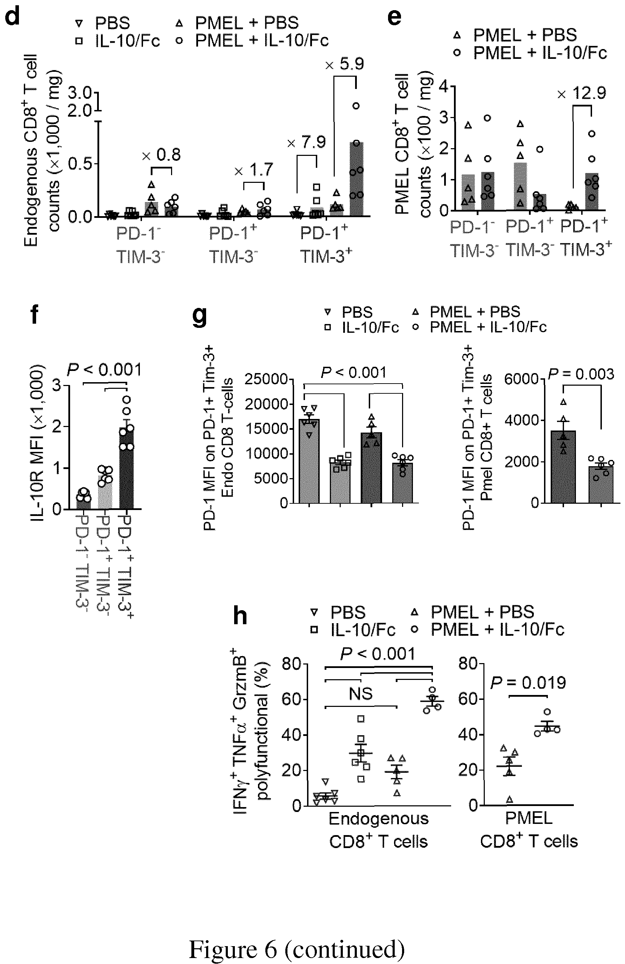

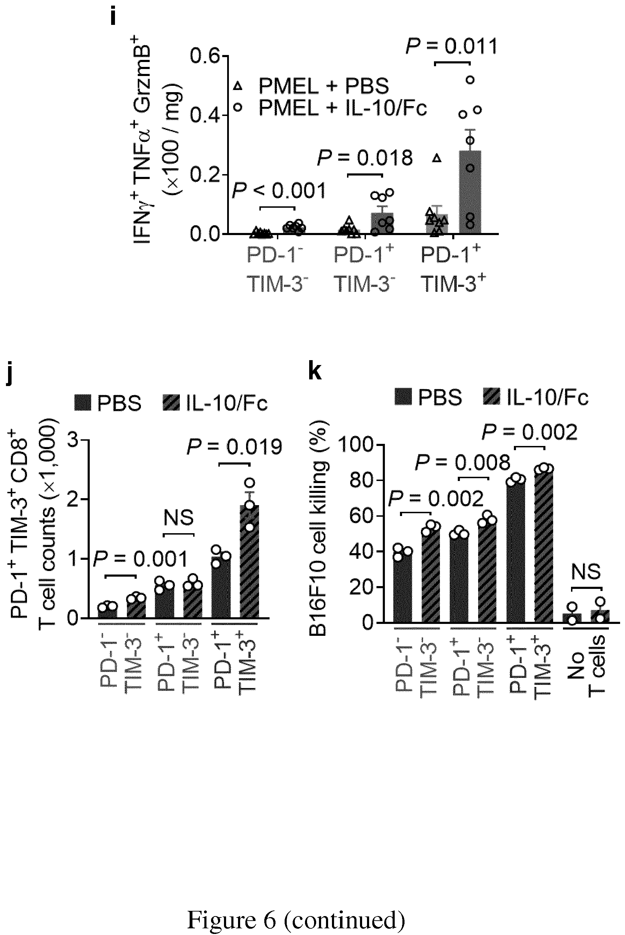

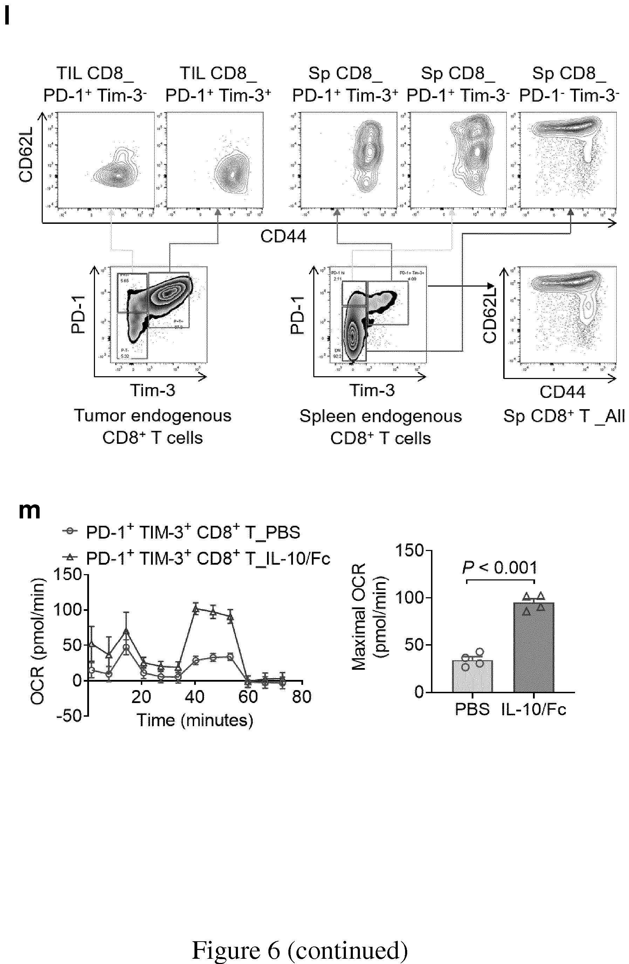

**Figure 6** shows IL-10/Fc specifically expands PD-1+ TIM-3+ cytotoxic T cells. B16F10 TILs were analyzed by flow cytometry as in Fig 5(a). **(a)** Total CD3⁺ T cells, total CD8⁺ T cells and CD4⁺ T cells density in tumor. **(b)** Immunofluorescence staining of CD3 for tumor sections from Fig 5(a). Scale bar: 50 µm. **(c)** Representative flow cytograms of endogenous CD8⁺ T cells and transferred Pmel CD8⁺ T cells. Statistical analysis of PD-1⁺ TIM-3⁺ percentages in endogenous CD8⁺ T cells and in transferred Pmel CD8⁺ T cells. **(d and e)** PD-1⁺ TIM-3⁺ endogenous CD8⁺ T cell **(d)** and PD-1⁺ TIM-3⁺ transferred Pmel CD8⁺ T cell **(e)** density in tumor. **(f)** IL-10R expression on different subpopulations of endogenous CD8⁺ T cells from PBS groups of B16F10 TILs. **(g)** PD-1 expression on PD-1⁺ TIM-3⁺ endogenous CD8⁺ T cells and on PD-1⁺ TIM-3⁺ transferred Pmel CD8⁺ T cells, **(h)** Percentage of polyfunctional (IPNγ⁺ TNFα⁺ Gramzyme B⁺) CD8 T cells in endogenous CD8⁺ T cells and in transferred Pmel CD8⁺ T cells. **(i)** Percentage of polyfunctional (IFNγ⁺ TNFα⁺ Gramzyme B⁺) CD8 T cells in endogenous PD-1⁺ TIM-3⁺ CD8⁺ T cells and in transferred Pmel PD-1⁺ TIM-3⁺ CD8⁺ T cells. **(j and k)** Sorted different subpopulations of CD8⁺ T cells depending on the expression of PD-1 and TIM-3 from Fig 2(h) co-cultured with B16F10 tumor cells for 2 days. CD8⁺ T cell counts **(j)** and B16F10 cell killing efficiency **(k)** were evaluated by flow cytometry. **(1)** Memory phenotype analyses (CD62L expression and CD44 expression) of PD-1⁺ TIM-3⁺ Endogenous CD8⁺ T cells from TILs and splenocytes. **(m)** Representative OCR trace and maximal OCR of PD-1⁺ TIM-3⁺ CD8⁺ T cells as in Fig 2(h). Data represent at least three independent experiments. Error bars indicate SEMs.

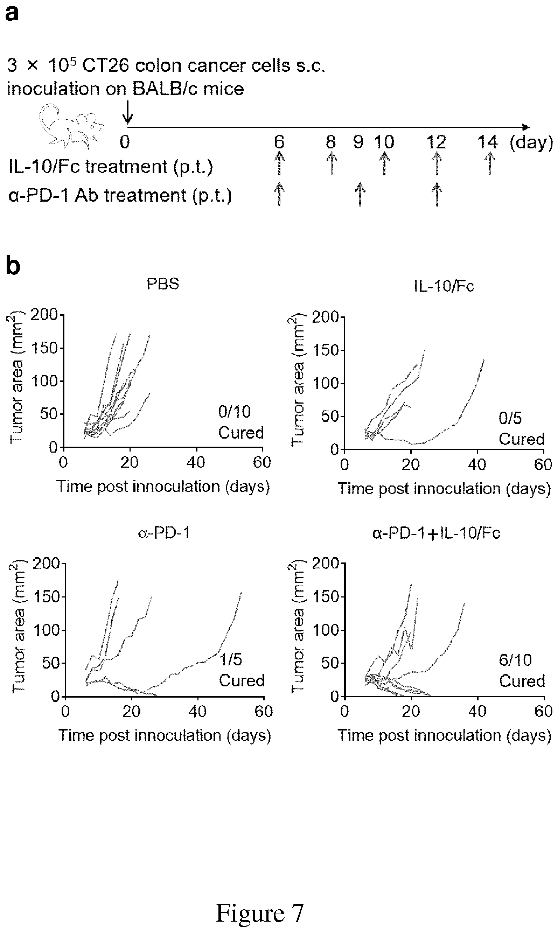

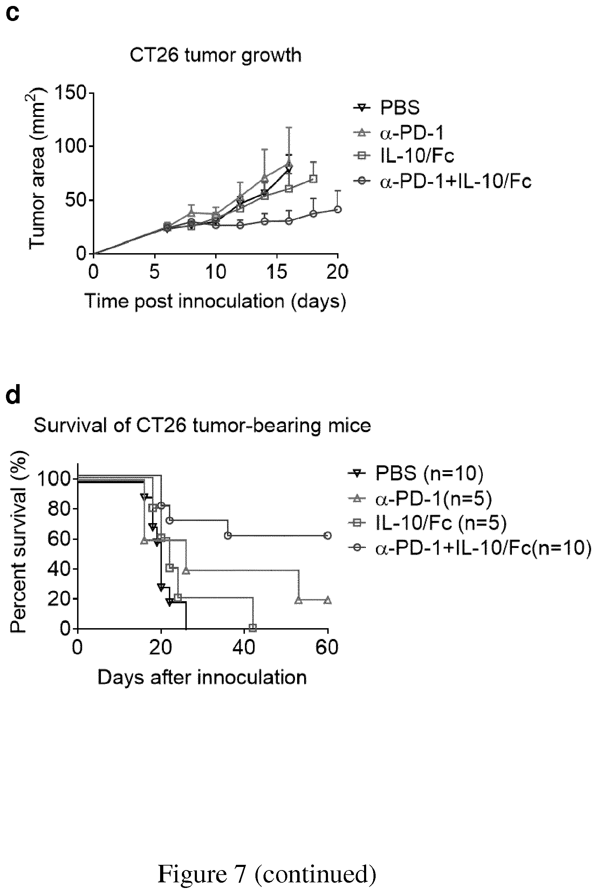

**Figure 7** shows IL-10/Fc potentiates checkpoint blockade therapy to eradicate established mouse colon tumors. (a) Experimental scheme of α-PD-1 and IL-10/Fc combination therapy for mouse CT26 colon cancer model. 3 x 105 CT26 mouse colon cancer cells were subcutaneously inoculated on BALB/c mice. α-PD-1 antibody (RMP-14) was given every three days as control. (b) Individual tumor growth curves of each group. (c) Tumor growth was monitored for mice treated as in (a). (d) Survival plot of mice treated as in (a). Data represent at least two independent experiments. Error bars indicate SEMs.

### Detailed description

As used herein, a "Fc fusion protein" relates to a Fc fusion protein (homodimer) comprising the sequence of a human IL-10 or a variant thereof and an IgG Fc fragment covalently linked together though a flexible hinge. According to one aspect, the IgG Fc fragment can be a Fc fragment from IgG1, IgG2, IgG3 or IgG4 isoform. IgG Fc fragments can be mutated for decreasing the antibody-dependent cell-mediated cytotoxicity (ADCC) such as described in Czajkowsky et al., 2012, EMBO Mol. Med, 1015-1028 or for increasing half-life or in vivo level of IgG as described in Zalevsky et al., 2010, Nat. Biotechnol. 28, 157-159*;* Vaccaro et al., 2005, Nat. Biotechnol. 23, 1283-1288 (e.g. IL-10/Fc).

As used herein, a "human IL-10 or a variant thereof" include sequences comprising the sequence of native human IL-10 and variants thereof such as described in Mumm et al., 2011, Cancer Cell, 20, 781-796*;* Guo, et al., 2012, Protein Expr. Purif. , 83, 152-156 (2012*);* Zheng et al., 1997, J. Immunol., 158, 4507-13*;* Qiao et al., 2019, Cancer Cell 35, 901-915.e4*.*

According to a particular aspect, Fc fusion proteins of the invention can be modified for extending its half-life *in vivo* by standard strategies, including pegylation (e.g. pegylation of the human IL-10 sequence or variant thereof: such as described in *Mumm et al., 2011, supra,* or the Fc domain of Fc fusion protein IL-10/Fc of the invention could also been replaced by antibodies or human serum albumin or variant thereof, such as described or reviewed in Qiao, et al., 2019, Cancer Cell 35, 901-915.e4*;* Kontermann, 2011, Curr. Opin. Biotechnol. 22, 868-876*).*

As used herein, a "flexible hinge" useful in the context of the invention can peptidic or non-peptidic. When the flexible hinge is of peptidic nature it generally contains between about 3 to 20 amino acids. For example, suitable hinge of the invention can be selected among those described in Klein et al., 2014, Protein Eng. Des. Sel. 27, 325-330*.* Non-peptidic flexible hinges can be selected among linkers described in Capon et al., 2011, Proc. Japan Acad. Ser. B Phys. Biol. Sci., 87, 603-616*.*

The term "variant", applied to a peptide or polypeptide, as referred to herein means a peptide or polypeptide substantially homologous to the referenced peptide sequence, but which has at least one amino acid different from that of the referenced sequence because of one or more amino acid deletion, insertion and/or substitution. Substantially homologous means a variant amino acid sequence which is identical to the referenced peptide sequence except for the deletion, insertion and/or substitution of 1, 2, 3, 4, 5 or 6 amino acid residues. In a more particular embodiment, a variant amino acid sequence is identical to the referenced peptide sequence except for the deletion and/or conservative substitution of 1, 2, 3, 4, 5 or 6 amino acid residues. The identity of two amino acid sequences can be determined by visual inspection and/or mathematical calculation, or more easily by comparing sequence information using known computer program used for sequence comparison such as Clustal package version 1.83. A variant may comprise a sequence having at least one conservatively substituted amino acid, meaning that a given amino acid residue is replaced by a residue having similar physicochemical characteristics. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Amino acid hydrophobicity can be found on the basis of known scales such as Kyte, et al, 1982, J. Mol. Biol., 157: 105- 131*;* Eisenberg, 1984, Ann. Rev. Biochem., 53: 595-623*.* Other such conservative substitutions, for example, substitutions of entire regions having similar hydrophobicity characteristics or secondary structure propensity, are well known (*Kyte, et al, 1982, supra*). For example, a "conservative amino acid substitution" may involve a substitution of a native amino acid residue with a non-native residue such that there is little or no effect on the polarity or charge of the amino acid residue at that position. Desired amino acid substitutions (whether conservative or non-conservative) can be determined by those skilled in the art at the time such substitutions are desired. Exemplary amino acid substitutions are presented in Table 1 below. The term "variant" also includes a peptide or polypeptide substantially homologous to the referenced peptide sequence, but which has an amino acid sequence different from that of the referenced sequence because one or more amino acids have been chemically modified or substituted by amino acids analogs. For example, non-natural residues can be introduced to enhance the pharmacological properties of peptide-based therapeutics (Geurink et al., 2013, J. Med. Chem., 56, 1262*;* Rand et al., 2012, Med. Chem. Commun, 3, 1282).

**Table 1**

| **Amino acids** | **Examples of « conservative » substitutions** |
|---|---|
| Ala (A) | Val, Leu, Ile |
| Arg (R) | Lys, Gln, Asn |
| Asn (N) | Gln |
| Asp (D) | Glu |
| Cys (C) | Ser, Ala |
| Gln (Q) | Asn |
| Glu (E) | Asp |
| Gly (G) | Pro, Ala |
| His (H) | Asn, Gln, Lys, Arg |
| Ile (I) | Leu, Val, Met, Ala, Phe, Norleucine |
| Leu (L) | Ile, Val, Met, Ala, Phe, Norleucine |
| Lys (K) | Arg, Gln, Asn |
| Met (M) | Leu, Ile, Phe |
| Phe (F) | Leu, Val, Ile, Ala, Tyr |
| Pro (P) | Ala, Gly |
| Ser (S) | Thr, Ala, Cys |
| Trp (W) | Phe, Tyr |
| Thr (T) | Ser |
| Tyr (Y) | Trp, Phe, Thr, Ser |
| Val (V) | Ile Met, Leu, Phe, Ala, Norleucine |

According to another particular embodiment, the sequence of the invention can be optionally acetylated at the N-terminus and/or amidated at the C-terminus.

According to another particular embodiment, the sequence of the invention can be optionally pegylated.

As used herein, "anti-cancer immunotherapy" refers to anti-cancer therapeutic strategies to effector activate immune cells which encompasses adoptive cellular therapy (ACT) and strategies to neutralize immunosuppressor mechanisms such as agents, in particular antibodies, against immune-checkpoint molecules such as cytotoxic T lymphocyte-associated protein 4 (CTLA-4) and programmed cell death protein 1 (PD1) such as described in Weiden et al., 2018, Nat. Rev. Immunol., 18, 212-219*.*

As used herein, "ACT immunotherapy" refers to a therapeutic technique where T-cells are collected from a patient's blood or tumour and grown in the laboratory. Once there are enough T-cells, they are given back to the patient to help their immune system to fight cancer cells.

Examples of ACT immunotherapies are listed under Fan et al., 2018, Theranostics, 8(20): 5784-5800*;* Rosenberg et al., 2008, Nat. Rev. Cancer 8, 299-308*;* Wang et al., 2014, Immunotherapy 6, 1265-1278*.*

As used herein, an "immune checkpoint inhibitor" refers to agents that antagonize or inhibit immune-checkpoint molecules and those can be selected from example among PD-1 inhibitors, PD-L1 inhibitors or CTLA-4 inhibitors or LAG-3 inhibitors such as described in Pardoll et al., 2012, Nat. Rev. Cancer 12, 252-264*;* Lee et al., 2019, Molecules 4, 1-16*.*

As used herein, "PD-1 inhibitors" include anti-PD-1 monoclonal antibodies, such as Keytruda and Opdivo such as described in *Lee et al., 2019, supra.*

As used herein, "PD-L1 inhibitors" include PD-L1 antibodies and PD-1-Ig fusion protein such as described in *Pardoll et al., 2012, supra.*

As used herein, "CTLA-4 inhibitors" include anti-CTLA-4 monoclonal antibodies, such as Ipilimumab and Tremelimumab such as described in *Pardoll et al., 2012, supra.*

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it such as a preventive early asymptomatic intervention; (b) inhibiting the disease, i.e., arresting its development; or relieving the disease, i.e., causing regression of the disease and/or its symptoms or conditions such as improvement or remediation of damage. In particular, the methods, uses, formulations and compositions according to the invention are useful for the prevention and/or treatment of a cancer.

The expression "solid tumour cancer" includes, lung cancer (small cell and non-small cell), breast cancer, ovarian cancer, cervical cancer, uterus cancer, head and neck cancer, glioblastoma, hepatocellular carcinoma, colon cancer, rectal cancer, colorectal carcinoma, kidney cancer, prostate cancer, gastric cancer, bronchus cancer, pancreatic cancer, urinary bladder cancer, hepatic cancer and brain cancer or skin cancer, in particular melanoma.

The term "subject" as used herein refers to mammals. For examples, mammals contemplated by the present invention include human, primates, domesticated animals such as cattle, sheep, pigs, horses, laboratory rodents, other pets and the like.

The term "effective amount" as used herein refers to an amount of at least one compound of the invention or a pharmaceutical formulation thereof according to the invention that elicits the biological or medicinal response in a tissue, system, animal or human that is being sought. In one embodiment, the effective amount is a "therapeutically effective amount" for the alleviation of the symptoms of the disease or condition being treated. In another embodiment, the effective amount is a "prophylactically effective amount" for prophylaxis of the symptoms of the disease or condition being prevented. The term also includes herein the amount of compound of the invention sufficient to reduce the progression of the disease, notably to reduce or inhibit the progression of a cancer disorder and thereby elicit the response being sought (i.e. an "effective amount"). Typically, an effective amount can be used to inhibit the growth of cancer cells, i.e. any slowing of the rate of cancer cell proliferation and/or migration, arrest of cancer cell proliferation and/or migration, or killing of cancer cells, such that the rate of cancer cell growth is reduced in comparison with the observed or predicted rate of growth of an untreated control cancer cell. The term "inhibits growth" can also refer to a reduction in size or disappearance of a cancer cell or tumor, as well as to a reduction in its metastatic potential. Preferably, such an inhibition at the cellular level may reduce the size, defer the growth, reduce the aggressiveness, or prevent or inhibit metastasis of a cancer in a patient. Those skilled in the art can readily determine, by any of a variety of suitable indicia, whether cancer cell growth is inhibited.

The term "efficacy" of a treatment according to the invention can be measured based on changes in the course of disease in response to a use or a method according to the invention. According to a particular embodiment, the efficacy can be measured through the measuring of the elicited immune response against cancer cells such as by analyzing tumor-specific T cells or by assessing cancer cell death and/or inhibition of tumor growth, progression and dissemination, reduction of tumour volume, and/or an increase of progression free survival time and/or increased health and well-being of the subject (e.g. repressing a cancer). The efficacy of a treatment of a cancer according to the invention can be measured by an inhibition of cancer cell growth evidenced for example by an arrest of cancer cells in a particular phase of the cell cycle, e.g., arrest at the G2/M phase of the cell cycle. Inhibition of cancer cell growth can also be evidenced using well known imaging methods such as magnetic resonance imaging, computerized axial tomography, PET, SPECT, photo-acoustic imaging, X-rays and fluorescence imaging/detection. Cancer cell growth can also be determined indirectly, for example by determining the levels of circulating carcino-embryonic antigen, prostate specific antigen or other cancer- specific antigens that are correlated with cancer cell growth.

In particular, efficacy of a combined treatment according to the invention can be assessed by expansion of tumor infiltrating CD8+ T cells, reduction of tumour size, disappearance of tumour, survival of tumor bearing mice or of any biomarker relevant for a cancer type.

"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. The prodrug is a derivative of the compound according to the invention and presenting anti-tumor activity that has a chemically or metabolically decomposable group, and a compound that may be converted into a pharmaceutically active compound *in vivo* under physiological conditions.

The term "pharmaceutical formulation" refers to preparations which are in such a form as to permit biological activity of the active ingredient(s) to be unequivocally effective and which contain no additional component which would be toxic to subjects to which the said formulation would be administered.

### Fusion proteins IL-10/Fc according to the invention and methods of preparation thereof

Fc fusion proteins (homodimers) suitable for use in the context of the invention are described herein.

According to a particular aspect, a Fc fusion protein (IL-10/Fc) according to the invention is a polypeptide comprising an immunoglobulin IgG Fc domain and a heterologous polypeptide a comprising a sequence of a human IL-10 or a variant thereof, wherein the heterologous polypeptide is covalently linked to the N-terminus or the C-terminus of the Fc domain by a polypeptide linker (e.g. a flexible hinge).

According to a particular aspect, the sequence of a Fc fusion protein according to the invention comprises the sequence of a human IL-10, for example wherein said sequence is of **SEQ ID NO: 1** or a variant thereof.

According to a particular aspect, the sequence of a Fc fusion protein according to the invention comprises the sequence of an IgG Fc fragment, wherein said IgG Fc fragment is a IgG1 Fc fragment or a variant thereof, in particular a non-cytolytic IgG1 Fc. For example, a fusion protein IL-10/Fc comprises an IgG Fc fragment of **SEQ ID NO: 2** or a variant thereof *(Zheng et al., 1997, supra;* Sazinsky et al., 2008, Proc. Natl. Acad. Sci. U. S. A. 105, 20167-20172*).*

According to a particular aspect, the sequence of a Fc fusion protein according to the invention comprises a flexible hinge selected from **SEQ ID NO: 3** and a sequence GGS or a variant thereof (*Klein et al., 2014, supra*).

According to a particular aspect, the sequence a Fc fusion protein according to the invention comprises a sequence of SEQ ID NO: 4 or a variant thereof.

According to a particular aspect, a Fc fusion protein IL-10/Fc comprises a sequence of a fusion protein IL-10/Fc described in *Guo et al., 2012, supra* or in Zheng et al., 1997, J. Immunol., 158, 4507-4513 or in Steele et al., 1995, J. Immunol., 154, 5590-5600 or variants or fragments thereof.

The Fc fusion proteins IL-10/Fc can be prepared as described herein or as described in *Guo et al., 2012, supra* or in Zheng *et al., 1997, supra* or in *Steele et al., 1995, supra.*

### Compositions according to the invention

The invention provides pharmaceutical or therapeutic agents as compositions and methods for treating a subject, preferably a mammalian subject, and most preferably a human patient who is suffering from a medical disorder, and in particular a cancer, in particular a solid tumor cancer.

In one embodiment, the invention provides a pharmaceutical composition containing at least one compound of the invention and a pharmaceutically acceptable carrier, diluent or excipient thereof.

Agent of the invention or formulations thereof may be administered as a pharmaceutical formulation, which can contain one or more agents according to the invention in any form described herein. The compositions according to the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use by injection or continuous infusion. Injectable compositions are typically based upon injectable sterile saline or phosphate-buffered saline or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Compositions of this invention may be liquid formulations including, but not limited to aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agents include, but are not limited to, sorbitol syrup, methylcellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.

Compositions of this invention may also be formulated as a depot preparation, which may be administered by implantation or by intramuscular injection.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maize starch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art.

According to a more particular aspect is provided a composition, wherein the sequence of said fusion protein comprises a sequence of **SEQ ID NO: 4** or a variant thereof.

The compounds of this invention can also be administered in sustained release forms or from sustained release drug delivery systems.

According to a particular embodiment, compositions according to the invention are for intravenous use.

According to a particular embodiment, compositions according to the invention are for intraperitoneal use.

According to a particular embodiment, compositions according to the invention are for intratumoral use.

In another particular aspect, the compositions according to the invention are adapted for delivery by repeated administration.

According to a particular embodiment, compositions of the invention are veterinary compositions.

Further materials as well as formulation processing techniques and the like are set out in Part 5 of Remington's "The Science and Practice of Pharmacy", 22nd Edition, 2012, University of the Sciences in Philadelphia, Lippincott Williams & Wilkins*,* which is incorporated herein by reference.

### Mode of administration

Compounds and formulations thereof according to this invention may be administered in any manner including parenterally, intravenously, intra-tumorally, intrathecally, transmucosally, intranasally, rectally, or combinations thereof. Parenteral administration includes, but is not limited to, intravenous, intra-arterial, intra-peritoneal, subcutaneous and intramuscular. The compositions of this invention may also be administered in the form of an implant, which allows slow release of the compositions as well as a slow controlled i.v. infusion.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combination

According to one aspect, compounds of the invention are to be administered in further combination with at least one therapeutic strategy useful in the prevention and/or treatment of a cancer, in particular an anti-cancer immunotherapy such as ACT therapy or immune checkpoint blockade therapy.

According to another particular aspect, the compounds of the invention are to be administered in combination with ACT therapy.

According to another particular aspect, the compounds of the invention are to be administered in combination with at least one immune checkpoint inhibitor.

According to one aspect is provided a pharmaceutical composition comprising at least one Fc fusion protein IL-10/Fc and a pharmaceutically acceptable carrier, diluent or excipient thereof and at least one agent useful in ACT therapy.

According to one aspect is provided a pharmaceutical composition comprising at least one Fc fusion protein IL-10/Fc and a pharmaceutically acceptable carrier, diluent or excipient thereof and at least one immune checkpoint inhibitor.

The invention encompasses the administration of a compound of the invention or a formulation thereof wherein it is administered to a subject prior to, simultaneously or sequentially with other therapeutic regimens or co-agents useful for preventing, treating, and/or stabilizing a cancer such as anti-cancer treatments.

A compound of the invention or a formulation thereof according to the invention that is administered simultaneously with said co-agents can be administered in the same or different composition(s) and by the same or different route(s) of administration.

According to one embodiment, is provided a pharmaceutical formulation comprising a compound of the invention combined with at least one co-agent useful for treating, and/or stabilizing, a neurodegenerative disorder and at least one pharmaceutically acceptable carrier.

### Use of Fc fusion proteins IL-10/Fc according to the invention

The invention provides a Fc fusion protein (IL-10/Fc) for use in the prevention and/or treatment of a cancer.

According to a particular aspect, is provided a Fc fusion protein (IL-10/Fc) for use in anti-cancer immunotherapy.

According to a more particular aspect, is provided a Fc fusion protein (IL-10/Fc) for use in combination with ACT immunotherapy, in particular based on TCR-T cells or CAR-T cells or TILs therapy.

### Methods according to the invention

According to another aspect, the invention provides a method of preventing or treating a cancer, in particular a solid tumor cancer.
According to another aspect, the invention provides a method of preventing and/or treating a lung cancer (small cell and non-small cell), breast cancer, prostate cancer, cancer, ovarian cancer, cervical cancer, uterus cancer, head and neck cancer, glioblastoma, hepatocellular carcinoma, colon cancer, rectal cancer, colorectal carcinoma, kidney cancer, gastric cancer, bronchus cancer, pancreatic cancer, urinary bladder cancer, hepatic cancer and brain cancer skin cancer.
According to another aspect, the invention provides a method of inducing immunity in a subject, said method comprising administering immune check-point blockades, in particular an anti-cancer immune check-point blockades in combination with a Fc fusion protein IL-10/Fc of the invention in a subject in need thereof.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims.
The invention having been described, the following examples are presented by way of illustration, and not limitation.

### Synthesis of compounds of the invention

The compounds of the invention can be prepared from readily available starting materials using the following general methods and procedures. It will be appreciated that where typical or preferred experimental conditions (i.e. culture or reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimization procedures.

### Patients

In an embodiment, patients according to the invention are suffering from any type of cancer. In an embodiment, patients according to the invention are suffering from any type of cancer at any stage, including non-metastatic and metastatic.
In a particular embodiment, patients according to the invention are suffering from lung cancer (small cell and non-small cell), breast cancer, prostate cancer, cancer, ovarian cancer, cervical cancer, uterus cancer, head and neck cancer, glioblastoma, hepatocellular carcinoma, colon cancer, rectal cancer, colorectal carcinoma, kidney cancer, gastric cancer, bronchus cancer, pancreatic cancer, urinary bladder cancer, hepatic cancer and brain cancer or skin cancer.
In a further particular embodiment, patients according to the invention are suffering from skin, breast, prostate, lung, pancreas, esophageal, hepatocellular, ovarian, colorectal and head and neck cancer and other solid tumors or any pre-malignant or malignant neoplasm.

In a further particular embodiment, patients according to the invention are suffering from melanoma.
In a further embodiment, subjects according to the invention are suffering from or at risk of suffering from a cancer.

Compounds, compositions and methods according to the invention are particularly useful for enhancing pyruvate dependent OXPHOS in CD8+ T cells and therefore in combination with immunotherapies such as those described therein.
References cited herein are hereby incorporated by reference in their entirety. The present invention is not to be limited in scope by the specific embodiments and drawings described herein, which are intended as single illustrations of individual aspects of the invention, and functionally equivalent methods and components are within the scope of the invention. The examples illustrating the invention are not intended to limit the scope of the invention in any way.

### EXAMPLES

The following studies are conducted to support the effectiveness of compounds of the invention according to the invention.

### Example 1: Preparation of a Fc fusion protein IL-10/Fc of the invention

A recombinant human IL-10 and IgG1 Fc fusion protein (IL-10/Fc of SEQ ID NO: 4) (**Fig**. **1a****)** which could cross-react with mouse or human IL-10 receptor (IL-10R) through IL-10 domain of the IL-10/Fc fusion protein was processed (Qiao et al., 2019, Cancer Cell 35, 901-915.e4*).* Therefore, the IL-10/Fc fusion protein could work on both mouse and human CD8+ T cells.

The IL-10/Fc fusion protein was expressed by HEK293 free style cells using the commercial mammalian expression vector, such as pcDNA3.1 or pSectag2A, carrying IL-10/Fc fusion gene as described before in *Guo et al., 2012, supra* or in Zheng et al., 1997, J. Immunol., 158, 4507-4513 or in Steele et al., 1995, J. Immunol., 154, 5590-5600 and then the culture supernatant containing IL-10/Fc was harvested by centrifuge after 7-day culture.

The IL-10/Fc fusion protein was first captured by Protein A column and then further purified by Superdex 200 increase column. The purified IL-10/Fc fusion protein was aliquoted and stored in -80 °C for stock. The purity of IL-10/Fc reached to 95% evidenced by SDS-PAGE and HPLC analysis. A HiTrap Protein A affinity chromatography column was used to capture the recombinant IL-10/Fc from the clarified expression supernatant filtered through 0.22-µm membranes, washed with 5 column volume binding buffer, and eluted with elution buffer (0.05 M sodium citrate, 0.3 M NaCl, pH 3.0). The eluted protein was immediately collected into neutralization buffer (1.0 M Tris-HCl, pH 10.0). The eluted protein was concentrated by 10 kDa membrane ultrafiltration (Vivaspin) and further purified with Superdex 200 increase size-exclusion chromatography at a flow rate of 1.0 mL/min with PBS, and the purified protein was aliquoted and stored at -80 °C.

### Example 2: Role of a fusion protein IL-10/Fc of the invention in IL-10-mediated metabolic reprograming

In a co-culture system of B16F10 mouse melanoma cells and activated Pmel CD8+ T-cell recognizing the gp100 cognate antigen, both basal and maximal oxygen consumption rates (OCR) of CD8+ T-cells were elevated upon treatment with the fusion protein IL-10/Fc of the invention whereas extracellular acidification rate (ECAR) remained unchanged **(****Fig. 2a****-c).** The ratio of OCR to EACR of CD8+ T-cells treated with the IL-10/Fc was markedly increased **(****Fig. 2d****),** suggesting that IL-10 signaling actively promotes T-cell oxidative phosphorylation (OXPHOS). As a result, T-cell counts and cytotoxicity was greatly enhanced **(****Fig. 2e** **and f).** Importantly, such metabolic reprograming effect was not observed in CD8+ T-cells without antigen stimulation **(****Fig. 2d****)** indicating the IL-10-mediated metabolic reprograming is TCR-signaling dependent. Similarly, it was found that the fusion protein IL-10/Fc also promoted OXPHOS of CD8+ T-cells during priming phase and enhanced T-cell proliferation **(****Fig. 1****).**

Next, several pathway-specific inhibitors such as 2-Deoxy-D-glucose, etomoxir, or UK5099 *(*Hildyard et al., 2005, Biochim. Biophys. Acta - Bioenerg., 1707, 221-230*)* were used to probe. 1.0 million/ml rested Pmel T cells were re-stimulated by soluble αCD3 tetramer (0.1 µg/ml) with or w/o indicated inhibitors for 2 days, and then T cell counts were analyzed by FACS assay, the molecular basis for IL-10/Fc in controlling CD8+ T cell metabolism **(****Fig. 2g****).** It was then found that inhibiting glucose uptake by 2-Deoxy-D-glucose (2DG) significantly impairs IL-10/Fc effect on CD8+ T cell proliferation **(****Fig. 2h****).** Limiting glucose impairs IL-10/Fc effect on CD8+ T cell proliferation (**Fig. 1e**). Interestingly, inhibition of fatty acid dependent-oxidation by ETO even further enhanced OXPHOS by IL-10/Fc **(****Fig. 2i****).** These results may indicate that the Fc fusion protein of the invention increased OXPHOS dependent on pyruvate oxidation rather than fatty acid β-oxidation, which was further confirmed by application of mitochondrial pyruvate carrier (MPC) inhibitor UK5099 to almost abrogate the effect of IL-10/Fc on T cell proliferation and metabolic reprograming **(****Fig. 2h** **and i).**
These data support that a fusion protein of the invention is able to reprograms T cells metabolism to promote T cell proliferation by increasing OXPHOSupon TCR stimulation through a pyruvate-dependent manner which may indicate that fusion protein of the invention could also promote tumor reactive CD8+ T cell expansion in the TME through reprograming T cells metabolism.

### Example 3: In-vivo anti tumoral effects of a fusion protein of the invention

Enhancing OXPHOS or inhibiting glycolytic metabolism in CD8+ T cells by various reagents promoted CD8+ T cell proliferation, memory development and antitumor function in TME *(*Zhang et al., 2017, Cancer Cell 32, 377-391.e9*;* Chowdhury et al., 2018, Cancer Immunol. Res. 6, 1375-1387*; Sukumar et al., 2013, 123, 4479-4488).* Based on the observed metabolic regulation function of the Fc fusion protein of the invention on CD8+ T-cells, investigating whether *in vivo* metabolic intervention of CD8 T-cells can be achieved with IL-10/Fc in view of enhancing the efficacy of adoptive T cell immunotherapies against solid tumors.
To overcome T cell exhaustion in the TME, *in vivo* anti-tumor effects of IL-10/Fc together with ACT of TCR transgenic CD8+ T cells (Pmel CD8+ T cell or OTI CD8+ T cells) or HER2 CAR-T cells in several tumor models, such as B16F10 (poorly immunogenic, highly aggressive mouse melanoma model), YUMM1.7-OVA (mouse melanoma model), or MC-38-HER2 (mouse colon carcinoma), respectively.
The treatment scheme in a B16F10 mouse melanoma model as depicted in **Fig. 3a** was carried out and it was observed that treatment with IL-10/Fc or ACT cells alone slightly controlled tumor growth over PBS group but most mice eventually succumbed with increased tumor burden **(****Fig. 4a****,** **Fig. 3b****-d).** Strikingly, combined treatment with IL-10/Fc and ACT therapy (ACT was performed by i.v. injection, and then followed by IL-10/Fc administration by intratumoral injection) induced significant regression of tumors, which led to durable cures in 90% of mice bearing B16F10 mouse melanoma **(****Fig. 4b****,** **Fig. 3e****).** Even with the decreased dose frequency of IL-10/Fc (**Fig. 3f****),** combination therapy of IL-10/Fc and ACT showed comparable efficacy as before **(****Fig. 3j****-g).** Notably, about 80% (11/14) of treated long-term survivors rejected a secondary B16F10 tumor cell challenge (0.1 million tumor cells per mouse) 3 months post the final injection **(****Fig. 4c****).**

To test the robustness of the combination therapy, IL-10/Fc treatment was combined with OT-I CD8+ T-cells in the treatment of an OVA-expressing mouse melanoma model (YUMM1.7-OVA) (Meeth et al., 2016, Pigment Cell Melanoma Res. 29, 590-597*;* Lane et al., 2018, J. Exp. Med. 215, 3057-3074*).* Tumor cells (1 million tumor cells per mouse) were inject s.c. and allowed to grow to high tumor burden (size > 60 mm2 or 150 mm3) (Pai et al., 2019, Immunity, 50, 477-492.e8*)* before the initiation of therapy. Administration of IL-10/Fc and ACT of OT-I CD8+ T-cells induced remarkable regression of tumor, while OT I CD8+ T cell ACT itself showed only transient tumor regression **(****Fig. 4d****).** Moreover, 60% (5/8) mice in IL-10/Fc and OT I CD8+ T cell ACT treatment group were cured while no mouse was cured in ACT alone group **(****Fig. 4e****).**

A combination therapy was also tested with chimeric antigen receptor T (CAR-T) cells and *in vivo* anti-tumor experiments were carried out using MC-38, a murine colon adenocarcinoma that expresses human epidermal growth factor receptor 2 (HER2). MC-38 was transduced by use of a retroviral construct containing the human HER2 encoding sequence.
It was observed that IL-10/Fc and HER2 CAR-T cells strongly suppressed MC-38-HER2 tumor growth **(****Fig. 4f****),** and eventually cured 80% (4/5) of tumor bearing mice in combination therapy group whereas HER2 CAR-T cell itself conferred very weak therapeutic benefit and none of mice survived at the end of experiment **(****Fig. 4g****).**

Those data supports that a fusion protein of the invention is able to potentiate adoptive T cells therapy by promoting T cell mediated tumor regression in both syngeneic and xenograft mouse models with pre-established solid tumors to eradicate established tumors.

### Example 4: In-vivo role of a fusion protein of the invention on immune infiltration of tumors

To understand the capacity of IL-10/Fc to drastically improve the efficacy of ACT, immune infiltration of tumors was analysed in B16F10 tumor model **(****Fig. 5a****).**
B16F10 tumor cells (1 × 10⁶) were inoculated subcutaneously in C57B1/6 mice and allowed to establish tumor for 6 d. Mice were then received PBS, IL-10/Fc, single adoptive transfer of 5 × 10⁶ activated pmel-1 CD8+ T cells (ACT) or IL-10/Fc and ACT combination therapy (combo) on day 6. Mice were received 4 injections of IL-10/Fc or PBS as indicated in experimental scheme. Mice were then sacrificed on day 14 and TILs were analyzed by flow cytometry.

It was shown that IL-10/Fc significantly increased tumor infiltrating T cells, in particular CD8+ T cells, in TME **(****Fig. 6a****),** but not natural killer (NK) cells or dendritic cells (DCs) **(****Fig. 5b****),** in the group treated with combination therapy compared with ACT alone. The results were confirmed with immunofluorescence staining of tumor sections **(****Fig. 6b****).** Furthermore, IL-10/Fc significantly decreased the percentage of Treg and increased the ratio CD8+ / Treg cells in TME **(****Fig. 5c****).**

These data indicated that IL-10/Fc and ACT synergistically reshaped the immunocellular composition, especially CD8+ T cell, of the TME to enhance anti-tumor immunity. Besides, IL-10/Fc treatment also markedly enhances the polyfunctions of both endogenous and adoptively transferred Pmel CD8+ T cells in tumors **(****Fig. 6h****).**

In order to determine which subset of CD8+ T-cells responded to IL-10/Fc treatment and contributed to the remarkably enhanced efficacy, activation/exhaustion makers were stained on the tumor infiltrating CD8+ T-cells. TILs isolated from tumor tissues were stained by anti mouse CD45.2, CD4, CD8, PD-1, and TIM-3 florescent antibodies, and then analysed by FACS assay. It has been reported recently that the PD-1+TIM-3+CD8+T cells previously defined as exhausted are in fact a highly proliferating, clonal, and dynamically differentiating cell population within the human tumor microenvironment with high cytotoxicity (Miller et al., 2019, Nat. Immunol. 20, 326-336*;* Li et al., 2019, Cell, 176, 775-789.e18). These results motivated us to investigate the ability of metabolic reprograming effect by IL-10/Fc treatment on this specific subset, the PD-1+TIM-3+CD8+ T cells.
This specific population observed *in vivo* was obtained by using anti-CD3 TCR triggering *in vitro,* mimicking the persistent antigen stimulation *in vivo.* 1.0 million/ml rested Pmel T cells were re-stimulated by soluble αCD3 tetramer (0.1 µg/ml) for 2 days.

It was observed that IL-10/Fc treatment significantly increased the portion of PD-1+Tim-3+ subsets among CD8+ T cells with the similar phenotype of "exhausted T cell" **(****Fig. 6c****).**
Further analysis show that IL-10/Fc selectively expanded PD-1+Tim-3+ but not other subsets in both endogenous (5.9 fold) and adoptively transferred Pmel CD8+ T cells (12.9 fold) in tumors (**Fig. 6d** **and e**). Consistent with this observation, IL-10 receptor expression was highly upregulated on PD-1+Tim-3+CD8+ T subsets in the TME **(****Fig. 6f****).**
Although PD-1+Tim-3+ subset was conventionally considered as "exhausted T-cells", we noticed that this subset in the combination treatment group showed decreased PD-1 expression level in both endogenous and Pmel CD8+ T cells **(****Fig. 6g****),** which may indicate reinvigoration of effector functions. Consistent with speculation, ployfunctional effector cells among the PD-1+TIM-3+CD8+ T cell subsets were greatly increased with IL-10/Fc treatment **(****Fig. 6i****).** High percentage of the intratumoral PD-1+Tim-3+ CD8+ T cell exhibit high cytotoxicity and proliferative capacity **(****Fig. 6j** **and k).** Further analysis show that PD-1+ Tim-3+ CD8+ T cells acquire memory phenotype in central lymphoid organs, which may contribute to memory CD8+ T cell development **(****Fig. 6I****).**
Consistently, both the cell counts and killing efficiency of PD-1+Tim-3+CD8+ T cells was accordingly increased with treatment of IL-10/Fc in T cell and B16F10 co-culture system **(****Fig. 6j** **and k).**
As expected, the OCR of the PD-1+Tim-3+CD8+ T subset was significantly increased by treatment of IL-10/Fc **(****Fig. 6m****),** which indicated that the PD-1+Tim-3+CD8+ T subset preferentially used OXPHOS over glycolysis after metabolic reprograming by IL-10/Fc.

Together, these results demonstrate that metabolic intervention via the use of a Fc fusion protein of the invention effectively reinvigorated CD8+ TILs, in particular the tumor infiltrating PD-1+Tim-3+ CD8+ T cells (cytotoxic T cells) by increasing the OXPHOS, to reacquire/maintain cytokine secretion ability, proliferation potentials, as well as long-term memory formation, where the PD-1+TIM-3+CD8+ T cell subset was expanded to 12.9 and 5.9-fold higher number compared to mice without IL-10/Fc treatment in adoptively transferred and endogenous CD8+ T cell, respectively.
Similarly, IL-10/Fc also enhanced the performance of checkpoint blockade therapy against the established solid tumor in mice **(****Fig. 7****).** Combination therapy of IL-10/Fc and α-PD-1 antibody significantly inhibited the tumor growth and promoted the survival of CT-26 tumor bearing mice **(****Fig. 7b****-d).**

Therefore, a Fc fusion protein of the invention provides a novel metabolic intervention strategy for highly effective ACT cancer immunotherapy against solid tumors such as TCR-T, CAR-T as well as TILs isolated from tumor tissues for personized cancer immunotherapy.
Further, based on those data, the use of a Fc fusion protein of the invention is considered as a safe and potent possible enhancer for immune check-point blockade therapy.

### SEQUENCE LISTING

**SEQ ID NO: 1 human IL-10** 
SPGQGTQSENSCTHFPGNLPNMLRDLRDAFSRVKTFFQMKDQLDNLLLKESLLEDFK
GYLGCQALSEMIQFYLEEVMPQAENQDPDIKAHVNSLGENLKTLRLRLRRCHRFLPC
ENKSKAVEQVKNAFNKLQEKGIYKAMSEFDIFINYIEAYMTMKIRN
**SEQ ID NO: 2 non-cytolytic IgG1 Fc** 
APEVAGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPASIEKTISKAKGQPR
EPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGK
**SEQ ID NO: 3 - Flexible hinge 1**
   EPKSCDKTHTCPPCP
**SEQ ID NO: 4 - IL-10/Fc fusion protein 1**

SPGQGTQSENSCTHFPGNLPNMLRDLRDAFSRVKTFFQMKDQLDNLLLKESLLEDFK
GYLGCQALSEMIQFYLEEVMPQAENQDPDIKAHVNSLGENLKTLRLRLRRCHRFLPC
ENKSKAVEQVKNAFNKLQEKGIYKAMSEFDIFINYIEAYMTMKIRNEPKSCDKTHTC
PPCPAPEVAGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPASIEKTISKA
KGQPREPQVYTLPPSREEMTKNQVSLTCLVKGFYPSDIAVEWESNGQPENNYKTTPP
VLDSDGSFFLYSKLTVDKSRWQQGNVFSCSVMHEALHNHYTQKSLSLSPGK

## Claims

1. A Fc fusion protein for use in the prevention and/or treatment of a cancer, wherein said Fc fusion protein is a polypeptide comprising an immunoglobulin IgG Fc domain and a heterologous polypeptide a comprising a sequence of a human IL-10 or a variant thereof, wherein the heterologous polypeptide is covalently linked to the N-terminus or the C-terminus of the Fc domain by a polypeptide linker (e.g. a flexible hinge).

2. A fusion protein for use according to claim 1, wherein the sequence of said Fc fusion protein comprises the sequence of a human IL-10.

3. A fusion protein for use according to claim 2, wherein the sequence of a human IL-10 is of **SEQ ID NO: 1** or a variant thereof.

4. A fusion protein for use according to any one of claims 1 to 3, wherein the sequence of said fusion protein comprises the sequence of an IgG1 Fc fragment or a variant thereof.

5. A fusion protein for use according to claim 4, wherein the sequence of the said IgG1 Fc fragment or variant thereof comprises a sequence of **SEQ ID NO: 2** or a variant thereof.

6. A fusion protein for use according to any one of claims 1 to 5, wherein the sequence of said fusion protein comprises the sequence of a flexible hinge selected from **SEQ ID NO: 3** and a sequence GGS or a variant thereof.

7. A fusion protein for use according to any one of claims 1 to 6, wherein the sequence of said fusion protein comprises a sequence of **SEQ ID NO: 4** or a variant thereof.

8. A fusion protein for use according to any one of claims 1 to 7, wherein said use is anti-cancer immunotherapy.

9. A fusion protein for use according to claim 8, wherein said anticancer immunotherapy is selected from ACT therapy or immune checkpoint blockades.

10. A fusion protein for use according to claim 8 or 9, wherein ACT therapy is selected from TCR-T, CAR-T or TILs therapies.

11. A fusion protein for use according to any one of claims 1 to 10, wherein said cancer is a solid tumor cancer, such as lung cancer, breast cancer, ovarian cancer, cervical cancer, uterus cancer, head and neck cancer, glioblastoma, hepatocellular carcinoma, colon cancer, rectal cancer, colorectal carcinoma, kidney cancer, prostate cancer, gastric cancer, bronchus cancer, pancreatic cancer, urinary bladder cancer, hepatic cancer and brain cancer or skin cancer, in particular melanoma.

12. A pharmaceutical composition comprising at least one Fc fusion protein comprising an immunoglobulin IgG Fc domain and a heterologous polypeptide a comprising a sequence of a human IL-10 or a variant thereof, wherein the heterologous polypeptide is covalently linked to the N-terminus or the C-terminus of the Fc domain by a polypeptide linker (e.g. a flexible hinge) and a pharmaceutically acceptable carrier, diluent or excipient thereof and at least one agent useful in anti-cancer immunotherapy.

13. A pharmaceutical composition according to claim 12, wherein the sequence of said Fc fusion protein comprises a sequence of **SEQ ID NO: 4** or a variant thereof.

14. A pharmaceutical composition according to claims 12 or 13 for use in the treatment of cancer, in particular ACT therapy.

15. A pharmaceutical composition for use according to claim 14 wherein ACT therapy is selected from TCR-T, CAR-T or TILs therapies.
